# EUROPEAN PATENT SPECIFICATION

(11) **EP 3 541 383 B1**
(45) Date of publication and mention of the grant of the patent: **06.01.2021**
(21) Application number: 17804759.3
(22) Date of filing: 10.11.2017
(51) Int. Cl.: A61K 31/435, A61K 39/395, A61P 35/00

(54) **COMBINATION THERAPY FOR CANCER WITH EXON 14 SKIPPING MUTATION(S) OR EXON 14 SKIPPING PHENOTYPE**
KOMBINATIONSTHERAPIE ZUR KREBSBEHANDLUNG MIT EXON-14-SKIPPING MUTATION(EN) ODER EXON-14-SKIPPING PHÄNOTYP
POLYTHÉRAPIE DU CANCER PAR MUTATION(S) AVEC SAUT DE L'EXON 14 OU DU PHÉNOTYPE AVEC SAUT DE L'EXON 14

(30) Priority: 16.11.2016 US 201662422887 P; 08.09.2017 US 201762555779 P
(43) Date of publication of application: 25.09.2019
(73) Proprietor: Eli Lilly and Company, Indianapolis, IN 46285 (US)
(72) Inventor: YAN, Sau-Chi Betty, Indianapolis, Indiana 46206-6288 (US)
(74) Representative: J A Kemp LLP
(86) International application number: PCT/US2017/061028
(87) International publication number: WO 2018/093669

(56) References cited:
- SAU-CHI BETTY YAN ET AL: "Abstract #528: Evaluation of single agent merestinib (LY2801653) or emibetuzumab (LY2875358) and the combination in a xenograft tumor model bearing MET exon 14 skipping", PROCEEDINGS OF THE AMERICAN ASSOCIATION FOR CANCER RESEARCH, vol. 58, 1 April 2017 (2017-04-01), page 132, XP055390405,
- REUNGWETWATTANA THANYANAN ET AL: "The race to targetMETexon 14 skipping alterations in non-small cell lung cancer: The Why, the How, the Who, the Unknown, and the Inevitable", LUNG CANCER, vol. 103, 15 November 2016 (2016-11-15), pages 27-37, XP029863831, ISSN: 0169-5002, DOI: 10.1016/J.LUNGCAN.2016.11.011
- ASSUNTA SGAMBATO ET AL: "The c-Met inhibitors: a new class of drugs in the battle against advanced nonsmall-cell lung cancer.", CURRENT PHARMACEUTICAL DESIGN, vol. 18, no. 37, 1 January 2012 (2012-01-01), pages 6155-6168, XP055087133, ISSN: 1381-6128, DOI: 10.2174/138161212803582478
- L. LIU ET AL: "LY2875358, a Neutralizing and Internalizing Anti-MET Bivalent Antibody, Inhibits HGF-Dependent and HGF-Independent MET Activation and Tumor Growth", CLINICAL CANCER RESEARCH, vol. 20, no. 23, 17 September 2014 (2014-09-17), pages 6059-6070, XP055425191, US ISSN: 1078-0432, DOI: 10.1158/1078-0432.CCR-14-0543
- YAN S BETTY ET AL: "MET-targeting antibody (emibetuzumab) and kinase inhibitor (merestinib) as single agent or in combination in a cancer model bearingMETexon 14 skipping", INVESTIGATIONAL NEW DRUGS, MARTINUS NIJHOFF PUBLISHERS, BOSTON, US, vol. 36, no. 4, 29 November 2017 (2017-11-29), pages 536-544, XP036554632, ISSN: 0167-6997, DOI: 10.1007/S10637-017-0545-X [retrieved on 2017-11-29]

### SEQUENCE LISTING

### SEQUENCE LISTING

<110> Eli Lilly and Company
<120> COMBINATION THERAPY FOR CANCER WITH MET GENOMIC MUTATIONS RESULTING IN EXON 14 SKIPPING OR EXON 14 SKIPPING PHENOTYPE
<130> P21348A
<160> 14
<170> PatentIn version 3.5
<210> 1
   <211> 108
   <212> PRT
   <213> Artificial Sequence
<220>
   <223> Synthetic Construct
<400> 1
<210> 2
   <211> 324
   <212> DNA
   <213> Artificial Sequence
<220>
   <223> Synthetic Construct
<400> 2
   gacatccaga tgacccagtc tccatcctcc ctgtctgcat ctgtaggaga cagagtcacc      60
   atcacttgca gtgtcagctc aagtgtatcc tccatttact tgcactggta tcagcagaaa     120
   ccagggaaag cccctaagct cctgatctat agcacatcca acttggcttc tggagtccca     180
   tcaaggttca gtggcagtgg atctgggaca gatttcactc tcaccatcag cagtctgcaa     240
   cctgaagatt ttgcaactta ctactgtcaa gtctacagtg gttacccgct cacgttcggc     300
   ggagggacca aggtggagat caaa                                            324
<210> 3
   <211> 115
   <212> PRT
   <213> Artificial Sequence
<220>
   <223> Synthetic Construct
<400> 3
<210> 4
   <211> 342
   <212> DNA
   <213> Artificial Sequence
<220>
   <223> Synthetic Construct
<400> 4
   caggttcagc tggtgcagtc tggtgctgag gtgaagaagc ctggtgcctc agtgaaggtc      60
   tcctgcaagg cttctggtta cacctttacc gactactaca tgcactgggt gcgtcaggcc     120
   cctggtcaag gtcttgagtg gatgggtcgt gttaatccta accggagggg tactacctac     180
   aaccagaaat tcgagggccg tgtcaccatg accacagaca catccacgag cacagcctac     240
   atggagctgc gtagcctgcg ttctgacgac acggccgtgt attactgtgc gcgtgcgaac     300
   tggcttgact actggggcca gggcaccacc gtcaccgtct cc                        342
<210> 5
   <211> 215
   <212> PRT
   <213> Artificial Sequence
<220>
   <223> Synthetic Construct
<400> 5
   Asp Ile Gln Met Thr Gln Ser Pro Ser Ser Leu Ser Ala Ser Val Gly
   1               5                   10                  15
   Asp Arg Val Thr Ile Thr Cys Ser Val Ser Ser Ser Val Ser Ser Ile
               20                  25                  30
   Tyr Leu His Trp Tyr Gln Gln Lys Pro Gly Lys Ala Pro Lys Leu Leu
           35                  40                  45
   Ile Tyr Ser Thr Ser Asn Leu Ala Ser Gly Val Pro Ser Arg Phe Ser
       50                  55                  60
   Gly Ser Gly Ser Gly Thr Asp Phe Thr Leu Thr Ile Ser Ser Leu Gln
   65                  70                  75                  80
   Pro Glu Asp Phe Ala Thr Tyr Tyr Cys Gln Val Tyr Ser Gly Tyr Pro
                   85                  90                  95
   Leu Thr Phe Gly Gly Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 6
   <211> 645
   <212> DNA
   <213> Artificial Sequence
<220>
   <223> Synthetic Construct
<400> 6
   gacatccaga tgacccagtc tccatcctcc ctgtctgcat ctgtaggaga cagagtcacc        60
   atcacttgca gtgtcagctc aagtgtatcc tccatttact tgcactggta tcagcagaaa       120
   ccagggaaag cccctaagct cctgatctat agcacatcca acttggcttc tggagtccca       180
   tcaaggttca gtggcagtgg atctgggaca gatttcactc tcaccatcag cagtctgcaa       240
   cctgaagatt ttgcaactta ctactgtcaa gtctacagtg gttacccgct cacgttcggc       300
   ggagggacca aggtggagat caaacgaact gtggctgcac catctgtctt catcttcccg       360
   ccatctgatg agcagttgaa atctggaact gcctctgttg tgtgcctgct gaataacttc       420
   tatcccagag aggccaaagt acagtggaag gtggataacg ccctccaatc gggtaactcc       480
   caggagagtg tcacagagca ggacagcaag gacagcacct acagcctcag cagcaccctg       540
   acgctgagca aagcagacta cgagaaacac aaagtctacg cctgcgaagt cacccatcag       600
   ggcctgagct cgcccgtcac aaagagcttc aacaggggag agtgc                       645
<210> 7
   <211> 441
   <212> PRT
   <213> Artificial Sequence
<220>
   <223> Synthetic Construct
<400> 7
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Tyr Met His Trp Val Arg Gln Ala Pro Gly Gln Gly Leu Glu Trp Met
           35                  40                  45
   Gly Arg Val Asn Pro Asn Arg Arg Gly Thr Thr Tyr Asn Gln Lys Phe
       50                  55                  60
   Glu Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Ala Asn Trp Leu Asp Tyr Trp Gly Gln Gly Thr Thr Val Thr
               100                 105                 110
   Val Ser Ser Ala Ser Thr Lys Gly Pro Ser Val Phe Pro Leu Ala Pro
           115                 120                 125
   Cys Ser Arg Ser Thr Ser Glu Ser Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Lys Thr Tyr Thr Cys Asn Val Asp His Lys Pro Ser Asn Thr Lys
           195                 200                 205
   Val Asp Lys Arg Val Glu Ser Lys Tyr Gly Pro Pro Cys Pro Pro Cys
       210                 215                 220
   Pro Ala Pro Glu Ala Ala Gly Gly Pro Ser Val Phe Leu Phe Pro Pro
   225                 230                 235                 240
   Lys Pro Lys Asp Thr Leu Met Ile Ser Arg Thr Pro Glu Val Thr Cys
                   245                 250                 255
   Val Val Val Asp Val Ser Gln Glu Asp Pro Glu Val Gln Phe Asn Trp
               260                 265                 270
   Tyr Val Asp Gly Val Glu Val His Asn Ala Lys Thr Lys Pro Arg Glu
           275                 280                 285
   Glu Gln Phe Asn Ser Thr Tyr Arg Val Val Ser Val Leu Thr Val Leu
       290                 295                 300
   His Gln Asp Trp Leu Asn Gly Lys Glu Tyr Lys Cys Lys Val Ser Asn
   305                 310                 315                 320
   Lys Gly Leu Pro Ser Ser Ile Glu Lys Thr Ile Ser Lys Ala Lys Gly
                   325                 330                 335
   Gln Pro Arg Glu Pro Gln Val Tyr Thr Leu Pro Pro Ser Gln Glu Glu
               340                 345                 350
   Met Thr Lys Asn Gln Val Ser Leu Thr Cys Leu Val Lys Gly Phe Tyr
           355                 360                 365
   Pro Ser Asp Ile Ala Val Glu Trp Glu Ser Asn Gly Gln Pro Glu Asn
       370                 375                 380
   Asn Tyr Lys Thr Thr Pro Pro Val Leu Asp Ser Asp Gly Ser Phe Phe
   385                 390                 395                 400
   Leu Tyr Ser Arg Leu Thr Val Asp Lys Ser Arg Trp Gln Glu Gly Asn
                   405                 410                 415
   Val Phe Ser Cys Ser Val Met His Glu Ala Leu His Asn His Tyr Thr
               420                 425                 430
   Gln Lys Ser Leu Ser Leu Ser Leu Gly
           435                 440
<210> 8
   <211> 1323
   <212> DNA
   <213> Artificial Sequence
<220>
   <223> Synthetic Construct
<400> 8
   caggttcagc tggtgcagtc tggtgctgag gtgaagaagc ctggtgcctc agtgaaggtc      60
   tcctgcaagg cttctggtta cacattcact gactactaca tgcactgggt gcgtcaggcc     120
   cctggtcaag gtcttgagtg gatgggtcgt gttaatccta accggagggg tactacctac     180
   aaccagaaat tcgagggccg tgtcaccatg accacagaca catccacgag cacagcctac     240
   atggagctgc gtagcctgcg ttctgacgac acggccgtgt attactgtgc gcgtgcgaac     300
   tggcttgact actggggcca gggcaccacc gtcaccgtct cctccgcctc caccaagggc     360
   ccatcggtct tcccgctagc gccctgctcc aggagcacct ccgagagcac agccgccctg       420
   ggctgcctgg tcaaggacta cttccccgaa ccggtgacgg tgtcgtggaa ctcaggcgcc       480
   ctgaccagcg gcgtgcacac cttcccggct gtcctacagt cctcaggact ctactccctc       540
   agcagcgtgg tgaccgtgcc ctccagcagc ttgggcacga agacctacac ctgcaacgta       600
   gatcacaagc ccagcaacac caaggtggac aagagagttg agtccaaata tggtccccca       660
   tgcccaccct gcccagcacc tgaggccgcc gggggaccat cagtcttcct gttcccccca       720
   aaacccaagg acactctcat gatctcccgg acccctgagg tcacgtgcgt ggtggtggac       780
   gtgagccagg aagaccccga ggtccagttc aactggtacg tggatggcgt ggaggtgcat       840
   aatgccaaga caaagccgcg ggaggagcag ttcaacagca cgtaccgtgt ggtcagcgtc       900
   ctcaccgtcc tgcaccagga ctggctgaac ggcaaggagt acaagtgcaa ggtctccaac       960
   aaaggcctcc cgtcctccat cgagaaaacc atctccaaag ccaaagggca gccccgagag      1020
   ccacaggtgt acaccctgcc cccatcccag gaggagatga ccaagaacca ggtcagcctg      1080
   acctgcctgg tcaaaggctt ctaccccagc gacatcgccg tggagtggga aagcaatggg      1140
   cagccggaga acaactacaa gaccacgcct cccgtgctgg actccgacgg ctccttcttc      1200
   ctctacagca ggctaaccgt ggacaagagc aggtggcagg aggggaatgt cttctcatgc      1260
   tccgtgatgc atgaggctct gcacaaccac tacacacaga agagcctctc cctgtctctg      1320
   ggt                                                                    1323
<210> 9
   <211> 932
   <212> PRT
   <213> Homo sapiens
<400> 9
   Met Lys Ala Pro Ala Val Leu Ala Pro Gly Ile Leu Val Leu Leu Phe
   1               5                   10                  15
   Thr Leu Val Gln Arg Ser Asn Gly Glu Cys Lys Glu Ala Leu Ala Lys
               20                  25                  30
   Ser Glu Met Asn Val Asn Met Lys Tyr Gln Leu Pro Asn Phe Thr Ala
           35                  40                  45
   Glu Thr Pro Ile Gln Asn Val Ile Leu His Glu His His Ile Phe Leu
       50                  55                  60
   Gly Ala Thr Asn Tyr Ile Tyr Val Leu Asn Glu Glu Asp Leu Gln Lys
   65                  70                  75                  80
   Val Ala Glu Tyr Lys Thr Gly Pro Val Leu Glu His Pro Asp Cys Phe
                   85                  90                  95
   Pro Cys Gln Asp Cys Ser Ser Lys Ala Asn Leu Ser Gly Gly Val Trp
               100                 105                 110
   Lys Asp Asn Ile Asn Met Ala Leu Val Val Asp Thr Tyr Tyr Asp Asp
           115                 120                 125
   Gln Leu Ile Ser Cys Gly Ser Val Asn Arg Gly Thr Cys Gln Arg His
       130                 135                 140
   Val Phe Pro His Asn His Thr Ala Asp Ile Gln Ser Glu Val His Cys
   145                 150                 155                 160
   Ile Phe Ser Pro Gln Ile Glu Glu Pro Ser Gln Cys Pro Asp Cys Val
                   165                 170                 175
   Val Ser Ala Leu Gly Ala Lys Val Leu Ser Ser Val Lys Asp Arg Phe
               180                 185                 190
   Ile Asn Phe Phe Val Gly Asn Thr Ile Asn Ser Ser Tyr Phe Pro Asp
           195                 200                 205
   His Pro Leu His Ser Ile Ser Val Arg Arg Leu Lys Glu Thr Lys Asp
       210                 215                 220
   Gly Phe Met Phe Leu Thr Asp Gln Ser Tyr Ile Asp Val Leu Pro Glu
   225                 230                 235                 240
   Phe Arg Asp Ser Tyr Pro Ile Lys Tyr Val His Ala Phe Glu Ser Asn
                   245                 250                 255
   Asn Phe Ile Tyr Phe Leu Thr Val Gln Arg Glu Thr Leu Asp Ala Gln
               260                 265                 270
   Thr Phe His Thr Arg Ile Ile Arg Phe Cys Ser Ile Asn Ser Gly Leu
           275                 280                 285
   His Ser Tyr Met Glu Met Pro Leu Glu Cys Ile Leu Thr Glu Lys Arg
       290                 295                 300
   Lys Lys Arg Ser Thr Lys Lys Glu Val Phe Asn Ile Leu Gln Ala Ala
   305                 310                 315                 320
   Tyr Val Ser Lys Pro Gly Ala Gln Leu Ala Arg Gln Ile Gly Ala Ser
                   325                 330                 335
   Leu Asn Asp Asp Ile Leu Phe Gly Val Phe Ala Gln Ser Lys Pro Asp
               340                 345                 350
   Ser Ala Glu Pro Met Asp Arg Ser Ala Met Cys Ala Phe Pro Ile Lys
           355                 360                 365
   Tyr Val Asn Asp Phe Phe Asn Lys Ile Val Asn Lys Asn Asn Val Arg
       370                 375                 380
   Cys Leu Gln His Phe Tyr Gly Pro Asn His Glu His Cys Phe Asn Arg
   385                 390                 395                 400
   Thr Leu Leu Arg Asn Ser Ser Gly Cys Glu Ala Arg Arg Asp Glu Tyr
                   405                 410                 415
   Arg Thr Glu Phe Thr Thr Ala Leu Gln Arg Val Asp Leu Phe Met Gly
               420                 425                 430
   Gln Phe Ser Glu Val Leu Leu Thr Ser Ile Ser Thr Phe Ile Lys Gly
           435                 440                 445
   Asp Leu Thr Ile Ala Asn Leu Gly Thr Ser Glu Gly Arg Phe Met Gln
       450                 455                 460
   Val Val Val Ser Arg Ser Gly Pro Ser Thr Pro His Val Asn Phe Leu
   465                 470                 475                 480
   Leu Asp Ser His Pro Val Ser Pro Glu Val Ile Val Glu His Thr Leu
                   485                 490                 495
   Asn Gln Asn Gly Tyr Thr Leu Val Ile Thr Gly Lys Lys Ile Thr Lys
               500                 505                 510
   Ile Pro Leu Asn Gly Leu Gly Cys Arg His Phe Gln Ser Cys Ser Gln
           515                 520                 525
   Cys Leu Ser Ala Pro Pro Phe Val Gln Cys Gly Trp Cys His Asp Lys
       530                 535                 540
   Cys Val Arg Ser Glu Glu Cys Leu Ser Gly Thr Trp Thr Gln Gln Ile
   545                 550                 555                 560
   Cys Leu Pro Ala Ile Tyr Lys Val Phe Pro Asn Ser Ala Pro Leu Glu
                   565                 570                 575
   Gly Gly Thr Arg Leu Thr Ile Cys Gly Trp Asp Phe Gly Phe Arg Arg
               580                 585                 590
   Asn Asn Lys Phe Asp Leu Lys Lys Thr Arg Val Leu Leu Gly Asn Glu
           595                 600                 605
   Ser Cys Thr Leu Thr Leu Ser Glu Ser Thr Met Asn Thr Leu Lys Cys
       610                 615                 620
   Thr Val Gly Pro Ala Met Asn Lys His Phe Asn Met Ser Ile Ile Ile
   625                 630                 635                 640
   Ser Asn Gly His Gly Thr Thr Gln Tyr Ser Thr Phe Ser Tyr Val Asp
                   645                 650                 655
   Pro Val Ile Thr Ser Ile Ser Pro Lys Tyr Gly Pro Met Ala Gly Gly
               660                 665                 670
   Thr Leu Leu Thr Leu Thr Gly Asn Tyr Leu Asn Ser Gly Asn Ser Arg
           675                 680                 685
   His Ile Ser Ile Gly Gly Lys Thr Cys Thr Leu Lys Ser Val Ser Asn
       690                 695                 700
   Ser Ile Leu Glu Cys Tyr Thr Pro Ala Gln Thr Ile Ser Thr Glu Phe
   705                 710                 715                 720
   Ala Val Lys Leu Lys Ile Asp Leu Ala Asn Arg Glu Thr Ser Ile Phe
                   725                 730                 735
   Ser Tyr Arg Glu Asp Pro Ile Val Tyr Glu Ile His Pro Thr Lys Ser
               740                 745                 750
   Phe Ile Ser Gly Gly Ser Thr Ile Thr Gly Val Gly Lys Asn Leu Asn
           755                 760                 765
   Ser Val Ser Val Pro Arg Met Val Ile Asn Val His Glu Ala Gly Arg
       770                 775                 780
   Asn Phe Thr Val Ala Cys Gln His Arg Ser Asn Ser Glu Ile Ile Cys
   785                 790                 795                 800
   Cys Thr Thr Pro Ser Leu Gln Gln Leu Asn Leu Gln Leu Pro Leu Lys
                   805                 810                 815
   Thr Lys Ala Phe Phe Met Leu Asp Gly Ile Leu Ser Lys Tyr Phe Asp
               820                 825                 830
   Leu Ile Tyr Val His Asn Pro Val Phe Lys Pro Phe Glu Lys Pro Val
           835                 840                 845
   Met Ile Ser Met Gly Asn Glu Asn Val Leu Glu Ile Lys Gly Asn Asp
   850 855 860
   Ile Asp Pro Glu Ala Val Lys Gly Glu Val Leu Lys Val Gly Asn Lys
   865 870 875 880
   Ser Cys Glu Asn Ile His Leu His Ser Glu Ala Val Leu Cys Thr Val
   885 890 895
   Pro Asn Asp Leu Leu Lys Leu Asn Ser Glu Leu Asn Ile Glu Trp Lys
   900 905 910
   Gln Ala Ile Ser Ser Thr Val Leu Gly Lys Val Ile Val Gln Pro Asp
   915 920 925
   Gln Asn Phe Thr
   930
<210> 10
   <211> 908
   <212> PRT
   <213> Homo sapiens
<400> 10
   Glu Cys Lys Glu Ala Leu Ala Lys Ser Glu Met Asn Val Asn Met Lys
   1 5 10 15
   Tyr Gln Leu Pro Asn Phe Thr Ala Glu Thr Pro Ile Gln Asn Val Ile
   20 25 30
   Leu His Glu His His Ile Phe Leu Gly Ala Thr Asn Tyr Ile Tyr Val
   35 40 45
   Leu Asn Glu Glu Asp Leu Gln Lys Val Ala Glu Tyr Lys Thr Gly Pro
   50 55 60
   Val Leu Glu His Pro Asp Cys Phe Pro Cys Gln Asp Cys Ser Ser Lys
   65 70 75 80
   Ala Asn Leu Ser Gly Gly Val Trp Lys Asp Asn Ile Asn Met Ala Leu
   85 90 95
   Val Val Asp Thr Tyr Tyr Asp Asp Gln Leu Ile Ser Cys Gly Ser Val
   100 105 110
   Asn Arg Gly Thr Cys Gln Arg His Val Phe Pro His Asn His Thr Ala
   115 120 125
   Asp Ile Gln Ser Glu Val His Cys Ile Phe Ser Pro Gln Ile Glu Glu
       130                 135                 140
   Pro Ser Gln Cys Pro Asp Cys Val Val Ser Ala Leu Gly Ala Lys Val
   145                 150                 155                 160
   Leu Ser Ser Val Lys Asp Arg Phe Ile Asn Phe Phe Val Gly Asn Thr
                   165                 170                 175
   Ile Asn Ser Ser Tyr Phe Pro Asp His Pro Leu His Ser Ile Ser Val
               180                 185                 190
   Arg Arg Leu Lys Glu Thr Lys Asp Gly Phe Met Phe Leu Thr Asp Gln
           195                 200                 205
   Ser Tyr Ile Asp Val Leu Pro Glu Phe Arg Asp Ser Tyr Pro Ile Lys
       210                 215                 220
   Tyr Val His Ala Phe Glu Ser Asn Asn Phe Ile Tyr Phe Leu Thr Val
   225                 230                 235                 240
   Gln Arg Glu Thr Leu Asp Ala Gln Thr Phe His Thr Arg Ile Ile Arg
                   245                 250                 255
   Phe Cys Ser Ile Asn Ser Gly Leu His Ser Tyr Met Glu Met Pro Leu
               260                 265                 270
   Glu Cys Ile Leu Thr Glu Lys Arg Lys Lys Arg Ser Thr Lys Lys Glu
           275                 280                 285
   Val Phe Asn Ile Leu Gln Ala Ala Tyr Val Ser Lys Pro Gly Ala Gln
       290                 295                 300
   Leu Ala Arg Gln Ile Gly Ala Ser Leu Asn Asp Asp Ile Leu Phe Gly
   305                 310                 315                 320
   Val Phe Ala Gln Ser Lys Pro Asp Ser Ala Glu Pro Met Asp Arg Ser
                   325                 330                 335
   Ala Met Cys Ala Phe Pro Ile Lys Tyr Val Asn Asp Phe Phe Asn Lys
               340                 345                 350
   Ile Val Asn Lys Asn Asn Val Arg Cys Leu Gln His Phe Tyr Gly Pro
           355                 360                 365
   Asn His Glu His Cys Phe Asn Arg Thr Leu Leu Arg Asn Ser Ser Gly
   370 375 380
   Cys Glu Ala Arg Arg Asp Glu Tyr Arg Thr Glu Phe Thr Thr Ala Leu
   385 390 395 400
   Gln Arg Val Asp Leu Phe Met Gly Gln Phe Ser Glu Val Leu Leu Thr
   405 410 415
   Ser Ile Ser Thr Phe Ile Lys Gly Asp Leu Thr Ile Ala Asn Leu Gly
   420 425 430
   Thr Ser Glu Gly Arg Phe Met Gln Val Val Val Ser Arg Ser Gly Pro
   435 440 445
   Ser Thr Pro His Val Asn Phe Leu Leu Asp Ser His Pro Val Ser Pro
   450 455 460
   Glu Val Ile Val Glu His Thr Leu Asn Gln Asn Gly Tyr Thr Leu Val
   465 470 475 480
   Ile Thr Gly Lys Lys Ile Thr Lys Ile Pro Leu Asn Gly Leu Gly Cys
   485 490 495
   Arg His Phe Gln Ser Cys Ser Gln Cys Leu Ser Ala Pro Pro Phe Val
   500 505 510
   Gln Cys Gly Trp Cys His Asp Lys Cys Val Arg Ser Glu Glu Cys Leu
   515 520 525
   Ser Gly Thr Trp Thr Gln Gln Ile Cys Leu Pro Ala Ile Tyr Lys Val
   530 535 540
   Phe Pro Asn Ser Ala Pro Leu Glu Gly Gly Thr Arg Leu Thr Ile Cys
   545 550 555 560
   Gly Trp Asp Phe Gly Phe Arg Arg Asn Asn Lys Phe Asp Leu Lys Lys
   565 570 575
   Thr Arg Val Leu Leu Gly Asn Glu Ser Cys Thr Leu Thr Leu Ser Glu
   580 585 590
   Ser Thr Met Asn Thr Leu Lys Cys Thr Val Gly Pro Ala Met Asn Lys
   595 600 605
   His Phe Asn Met Ser Ile Ile Ile Ser Asn Gly His Gly Thr Thr Gln
   610 615 620
   Tyr Ser Thr Phe Ser Tyr Val Asp Pro Val Ile Thr Ser Ile Ser Pro
   625                 630                 635                 640
   Lys Tyr Gly Pro Met Ala Gly Gly Thr Leu Leu Thr Leu Thr Gly Asn
                   645                 650                 655
   Tyr Leu Asn Ser Gly Asn Ser Arg His Ile Ser Ile Gly Gly Lys Thr
               660                 665                 670
   Cys Thr Leu Lys Ser Val Ser Asn Ser Ile Leu Glu Cys Tyr Thr Pro
           675                 680                 685
   Ala Gln Thr Ile Ser Thr Glu Phe Ala Val Lys Leu Lys Ile Asp Leu
       690                 695                 700
   Ala Asn Arg Glu Thr Ser Ile Phe Ser Tyr Arg Glu Asp Pro Ile Val
   705                 710                 715                 720
   Tyr Glu Ile His Pro Thr Lys Ser Phe Ile Ser Gly Gly Ser Thr Ile
                   725                 730                 735
   Thr Gly Val Gly Lys Asn Leu Asn Ser Val Ser Val Pro Arg Met Val
               740                 745                 750
   Ile Asn Val His Glu Ala Gly Arg Asn Phe Thr Val Ala Cys Gln His
           755                 760                 765
   Arg Ser Asn Ser Glu Ile Ile Cys Cys Thr Thr Pro Ser Leu Gln Gln
       770                 775                 780
   Leu Asn Leu Gln Leu Pro Leu Lys Thr Lys Ala Phe Phe Met Leu Asp
   785                 790                 795                 800
   Gly Ile Leu Ser Lys Tyr Phe Asp Leu Ile Tyr Val His Asn Pro Val
                   805                 810                 815
   Phe Lys Pro Phe Glu Lys Pro Val Met Ile Ser Met Gly Asn Glu Asn
               820                 825                 830
   Val Leu Glu Ile Lys Gly Asn Asp Ile Asp Pro Glu Ala Val Lys Gly
           835                 840                 845
   Glu Val Leu Lys Val Gly Asn Lys Ser Cys Glu Asn Ile His Leu His
       850                 855                 860
   Ser Glu Ala Val Leu Cys Thr Val Pro Asn Asp Leu Leu Lys Leu Asn
   865                 870                 875                 880
   Ser Glu Leu Asn Ile Glu Trp Lys Gln Ala Ile Ser Ser Thr Val Leu
   885 890 895
   Gly Lys Val Ile Val Gln Pro Asp Gln Asn Phe Thr
   900 905
<210> 11
   <211> 283
   <212> PRT
   <213> Homo sapiens
<400> 11
   Glu Cys Lys Glu Ala Leu Ala Lys Ser Glu Met Asn Val Asn Met Lys
   1 5 10 15
   Tyr Gln Leu Pro Asn Phe Thr Ala Glu Thr Pro Ile Gln Asn Val Ile
   20 25 30
   Leu His Glu His His Ile Phe Leu Gly Ala Thr Asn Tyr Ile Tyr Val
   35 40 45
   Leu Asn Glu Glu Asp Leu Gln Lys Val Ala Glu Tyr Lys Thr Gly Pro
   50 55 60
   Val Leu Glu His Pro Asp Cys Phe Pro Cys Gln Asp Cys Ser Ser Lys
   65 70 75 80
   Ala Asn Leu Ser Gly Gly Val Trp Lys Asp Asn Ile Asn Met Ala Leu
   85 90 95
   Val Val Asp Thr Tyr Tyr Asp Asp Gln Leu Ile Ser Cys Gly Ser Val
   100 105 110
   Asn Arg Gly Thr Cys Gln Arg His Val Phe Pro His Asn His Thr Ala
   115 120 125
   Asp Ile Gln Ser Glu Val His Cys Ile Phe Ser Pro Gln Ile Glu Glu
   130 135 140
   Pro Ser Gln Cys Pro Asp Cys Val Val Ser Ala Leu Gly Ala Lys Val
   145 150 155 160
   Leu Ser Ser Val Lys Asp Arg Phe Ile Asn Phe Phe Val Gly Asn Thr
   165 170 175
   Ile Asn Ser Ser Tyr Phe Pro Asp His Pro Leu His Ser Ile Ser Val
   180 185 190
   Arg Arg Leu Lys Glu Thr Lys Asp Gly Phe Met Phe Leu Thr Asp Gln
           195                 200                 205
   Ser Tyr Ile Asp Val Leu Pro Glu Phe Arg Asp Ser Tyr Pro Ile Lys
       210                 215                 220
   Tyr Val His Ala Phe Glu Ser Asn Asn Phe Ile Tyr Phe Leu Thr Val
   225                 230                 235                 240
   Gln Arg Glu Thr Leu Asp Ala Gln Thr Phe His Thr Arg Ile Ile Arg
                   245                 250                 255
   Phe Cys Ser Ile Asn Ser Gly Leu His Ser Tyr Met Glu Met Pro Leu
               260                 265                 270
   Glu Cys Ile Leu Thr Glu Lys Arg Lys Lys Arg
           275                 280
<210> 12
   <211> 212
   <212> PRT
   <213> Homo sapiens
<400> 12
   Ser Thr Lys Lys Glu Val Phe Asn Ile Leu Gln Ala Ala Tyr Val Ser
   1               5                   10                  15
   Lys Pro Gly Ala Gln Leu Ala Arg Gln Ile Gly Ala Ser Leu Asn Asp
               20                  25                  30
   Asp Ile Leu Phe Gly Val Phe Ala Gln Ser Lys Pro Asp Ser Ala Glu
           35                  40                  45
   Pro Met Asp Arg Ser Ala Met Cys Ala Phe Pro Ile Lys Tyr Val Asn
       50                  55                  60
   Asp Phe Phe Asn Lys Ile Val Asn Lys Asn Asn Val Arg Cys Leu Gln
   65                  70                  75                  80
   His Phe Tyr Gly Pro Asn His Glu His Cys Phe Asn Arg Thr Leu Leu
                   85                  90                  95
   Arg Asn Ser Ser Gly Cys Glu Ala Arg Arg Asp Glu Tyr Arg Thr Glu
               100                 105                 110
   Phe Thr Thr Ala Leu Gln Arg Val Asp Leu Phe Met Gly Gln Phe Ser
           115                 120                 125
   Glu Val Leu Leu Thr Ser Ile Ser Thr Phe Ile Lys Gly Asp Leu Thr
       130                 135                 140
   Ile Ala Asn Leu Gly Thr Ser Glu Gly Arg Phe Met Gln Val Val Val
   145                 150                 155                 160
   Ser Arg Ser Gly Pro Ser Thr Pro His Val Asn Phe Leu Leu Asp Ser
                   165                 170                 175
   His Pro Val Ser Pro Glu Val Ile Val Glu His Thr Leu Asn Gln Asn
               180                 185                 190
   Gly Tyr Thr Leu Val Ile Thr Gly Lys Lys Ile Thr Lys Ile Pro Leu
           195                 200                 205
   Asn Gly Leu Gly
       210
<210> 13
   <211> 214
   <212> PRT
   <213> Artificial Sequence
<220>
   <223> Synthetic Construct
<400> 13
   Glu Ile Gln Met Thr Gln Ser Pro Ala Ser Leu Ser Ala Ser Val Gly
   1               5                   10                  15
   Glu Thr Val Thr Ile Thr Cys Arg Ala Ser Glu Asn Ile Tyr Ser Tyr
               20                  25                  30
   Leu Ala Trp Tyr Gln Arg Lys Gln Gly Arg Ser Pro Gln Leu Leu Val
           35                  40                  45
   Tyr Asn Ala Lys Pro Leu Ala Glu Gly Val Pro Ser Arg Phe Ser Gly
       50                  55                  60
   Ser Gly Ser Gly Thr Gln Phe Ser Leu Lys Ile Asn Ser Leu Gln Pro
   65                  70                  75                  80
   Glu Asp Phe Gly Thr Tyr Tyr Cys Gln His His Tyr Gly Thr Pro Phe
                   85                  90                  95
   Thr Phe Gly Ser Gly Thr Arg Leu Glu Ile Lys Arg Ala Asp Ala Ala
               100                 105                 110
   Pro Thr Val Ser Ile Phe Pro Pro Ser Ser Glu Gln Leu Thr Ser Gly
           115                 120                 125
   Gly Ala Ser Val Val Cys Phe Leu Asn Asn Phe Tyr Pro Lys Asp Ile
       130                 135                 140
   Asn Val Lys Trp Lys Ile Asp Gly Ser Glu Arg Gln Asn Gly Val Leu
   145                 150                 155                 160
   Asn Ser Trp Thr Asp Gln Asp Ser Lys Asp Ser Thr Tyr Ser Met Ser
                   165                 170                 175
   Ser Thr Leu Thr Leu Thr Lys Asp Glu Tyr Glu Arg His Asn Ser Tyr
               180                 185                 190
   Thr Cys Glu Ala Thr His Lys Thr Ser Thr Ser Pro Ile Val Lys Ser
           195                 200                 205
   Phe Asn Arg Asn Glu Cys
       210
<210> 14
   <211> 443
   <212> PRT
   <213> Artificial Sequence
<220>
   <223> Synthetic Construct
<400> 14
   Glu Val Gln Leu Gln Gln Ser Gly Thr Val Leu Ala Arg Pro Gly Ala
   1               5                   10                  15
   Ser Val Lys Met Ser Cys Lys Ala Ser Gly Tyr Ser Phe Thr Ser Tyr
               20                  25                  30
   Trp Met Tyr Trp Val Lys Gln Arg Pro Gly Gln Gly Leu Glu Trp Ile
           35                  40                  45
   Gly Gly Phe His Pro Arg Asn Ser Gly Thr Asn Tyr Asn Gln Lys Phe
       50                  55                  60
   Lys Gly Lys Ala Lys Leu Thr Ala Val Thr Ser Ala Ser Thr Ala Tyr
   65                  70                  75                  80
   Met Glu Leu Ser Ser Leu Thr Asn Glu Asp Ser Ala Val Tyr Tyr Cys
                   85                  90                  95
   Thr Arg Gly Tyr Tyr Tyr Asp Gly Ser Phe Thr Tyr Trp Gly Gln Gly
               100                 105                 110
   Thr Leu Val Thr Val Ser Ala Ala Lys Thr Thr Pro Pro Ser Val Tyr
           115                 120                 125
   Pro Leu Ala Pro Gly Ser Ala Ala Gln Thr Asn Ser Met Val Thr Leu
       130                 135                 140
   Gly Cys Leu Val Lys Gly Tyr Phe Pro Glu Pro Val Thr Val Thr Trp
   145                 150                 155                 160
   Asn Ser Gly Ser Leu Ser Ser Gly Val His Thr Phe Pro Ala Val Leu
                   165                 170                 175
   Gln Ser Asp Leu Tyr Thr Leu Ser Ser Ser Val Thr Val Pro Ser Ser
               180                 185                 190
   Thr Trp Pro Ser Glu Thr Val Thr Cys Asn Val Ala His Pro Ala Ser
           195                 200                 205
   Ser Thr Lys Val Asp Lys Lys Ile Val Pro Arg Asp Cys Gly Cys Lys
       210                 215                 220
   Pro Cys Ile Cys Thr Val Pro Glu Val Ser Ser Val Phe Ile Phe Pro
   225                 230                 235                 240
   Pro Lys Pro Lys Asp Val Leu Thr Ile Thr Leu Thr Pro Lys Val Thr
                   245                 250                 255
   Cys Val Val Val Asp Ile Ser Lys Asp Asp Pro Glu Val Gln Phe Ser
               260                 265                 270
   Trp Phe Val Asp Asp Val Glu Val His Thr Ala Gln Thr Gln Pro Arg
           275                 280                 285
   Glu Glu Gln Phe Asn Ser Thr Phe Arg Ser Val Ser Glu Leu Pro Ile
       290                 295                 300
   Met His Gln Asp Trp Leu Asn Gly Lys Glu Phe Lys Cys Arg Val Asn
   305                 310                 315                 320
   Ser Ala Ala Phe Pro Ala Pro Ile Glu Lys Thr Ile Ser Lys Thr Lys
                   325                 330                 335
   Gly Arg Pro Lys Ala Pro Gln Val Tyr Thr Ile Pro Pro Pro Lys Glu
               340                 345                 350
   Gln Met Ala Lys Asp Lys Val Ser Leu Thr Cys Met Ile Thr Asp Phe
           355                 360                 365
   Phe Pro Glu Asp Ile Thr Val Glu Trp Gln Trp Asn Gly Gln Pro Ala
       370                 375                 380
   Glu Asn Tyr Lys Asn Thr Gln Pro Ile Met Asp Thr Asp Gly Ser Tyr
   385                 390                 395                 400
   Phe Val Tyr Ser Lys Leu Asn Val Gln Lys Ser Asn Trp Glu Ala Gly
                   405                 410                 415
   Asn Thr Phe Thr Cys Ser Val Leu His Glu Gly Leu His Asn His His
               420                 425                 430
   Thr Glu Lys Ser Leu Ser His Ser Pro Gly Lys
           435                 440

## Description

The present invention relates to combinations of a ligand neutralizing and MET receptor internalizing bivalent anti-human MET antibody, emibetuzumab, with an anti-human MET kinase inhibitor, merestinib, or a pharmaceutically acceptable salt thereof, and to uses of the combinations to treat certain disorders, such as lung cancer and gastric cancer, in patients with tumors bearing MET exon 14 skipping(s) or MET exon 14 skipping phenotype.

Overexpression and activation of MET tyrosine receptor kinase can be an oncogenic driver of tumor growth in many types of cancer. The MET signaling pathway regulates a wide variety of normal cellular functions that can be subverted to support neoplasia, including cell proliferation, survival, apoptosis, scattering and motility, invasion, and angiogenesis. MET over-expression (with or without gene amplification), aberrant autocrine or paracrine ligand production, and missense MET mutations are mechanisms that lead to activation of the MET pathway in tumors and are associated with poor prognostic outcome. Combinations of MET inhibitors and MET antibodies have been contemplated in the art. *See,* US 20140037625 and WO 2012031027.

Different genomic changes may occur in the intronic and/or exonic segments of MET and can lead to an alternatively spliced transcript of MET where exon 14 is skipped (i.e., exon 14 is largely or entirely deleted). MET exon 14 skipping mutations result in a protein missing the Y1003 phosphorylation site, the binding site for the ubiquitin ligase CBL, which targets MET for degradation. Additionally, a single point mutation at Y1003, D1002 or R1004 will also result in an inability of the ubiquitin ligase CBL to bind to the MET receptor without skipping of exon 14 (*i.e.,* MET exon 14 skipping phenotype). This results in a MET protein with increased stability and oncogenic potential. MET exon 14 skipping mutations or an exon 14 skipping phenotype has been observed in adenosquamous, adenocarcinoma, sarcomatoid, squamous cell, large cell, and small cell histologies. Specifically, MET exon 14 skipping has been detected in lung adeonocarcinoma, as well as in neuroblastoma, gastric, and colon cancer cell lines. Tumors with MET exon 14 skipping mutations have been reported to be responsive to treatment with MET inhibitors in clinical case reports and series.

MET exon 14 skipping mutation (s) or exon 14 skipping phenotype are targetable mutations in lung cancer and is reported in approximately 3 to 6 % of non-small cell lung cancer (NSCLC) patients. Lung cancer remains the third most prevalent cancer in the United States and is the leading cause of cancer death in both men and women throughout the world. The two main types of lung cancer are small cell lung cancer and NSCLC. The majority of patients with lung cancer have advanced and/or metastatic disease at diagnosis and the majority of patients treated with curative intent develop recurrence. These patients present with advanced, inoperable stage cancer for which there is no prospect of cure. Treatment is provided to improve symptoms, optimize quality of life, and prolong survival.

MET exon 14 skipping mutation(s) or MET exon 14 skipping phenotype are also targetable mutations in gastric cancer, a malignant tumor that originates in the stomach lining. Gastric cancers are classified according to the type of tissue from which they originate, with the most common type being adenocarcinoma and accounts for over 90% of all stomach cancers. Adenocarcinoma of the esophagus including carcinoma of the gastroesophageal junction (GEJ) is one of the fastest rising malignancies and is associated with a poor prognosis. Other forms of gastric cancer include lymphomas and sarcomas. Gastric cancer may be cured if it is found and treated at an early stage, but unfortunately, it is often found at a later stage.

There remains a need for the treatment of cancers with tumors bearing MET exon 14 skipping mutation(s) or MET exon 14 skipping phenotype. Tumors bearing MET exon 14 skipping mutation(s) or MET exon 14 skipping phenotype can have a response to MET inhibitors. Thus, a combination of an anti-human MET inhibitor, merestinib, or a pharmaceutically acceptable salt thereof, with an anti-human MET neutralizing and internalizing bivalent antibody, emibetuzumab, may provide a treatment option for cancer patients who have tumors bearing MET exon 14 skipping mutations or MET exon 14 skipping phenotype.

*N*-(3-Fluoro-4-(1-methyl-6-(1*H*-pyrazol-4-yl)-1*H*-indazol-5-yloxy)phenyl)-1-(4-fluorophenyl)-6-methyl-2-oxo-1,2-dihydropyridine-3-carboxamide (CAS # 1206799-15-6), also known as merestinib and represented by the structural formula (I) below, is a small molecule type II MET kinase inhibitor. Merestinib and methods of making and using this compound and pharmaceutically acceptable salts thereof including for the treatment of neoplastic diseases such as solid and non-solid tumors are disclosed in WO 2010/011538. Furthermore, merestinib is currently being evaluated in Phase 2 clinical studies for patients in NSCLC and solid tumors (see ClinicalTrials.gov NCT02920996).

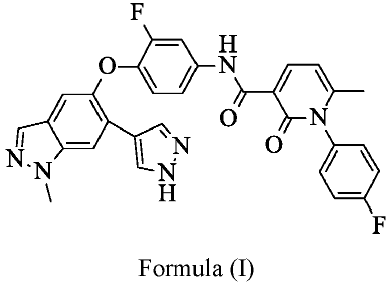

Anti-human MET monoclonal antibodies, including, but not limited to, C8-H241 antibodies, were previously disclosed in WO 2010/059654. Emibetuzumab is a C8-H241-IgG4 antibody comprising: two light chains, each of whose amino acid sequence is that given in SEQ ID NO: 5, and two heavy chains, each of whose amino acid sequence is that given in SEQ ID NO: 7 (see, WHO Drug Information, Proposed International Nonproprietary Names (INN) List 111, Volume 28, No. 2, July 2014). Emibetuzumab has high neutralization and internalization activities resulting in inhibition of both HGF-dependent and HGF-independent (e.g., MET amplified) MET pathway activation and tumor growth in xenograft models. Emibetuzumab is currently being evaluated in Phase 2 clinical studies in combination with erlotinib in NSCLC patients (see, ClinicalTrials.gov NCT01900652, NCT01897480).

The present invention provides treatment of cancer with tumors bearing MET exon 14 skipping mutation(s) or MET exon 14 skipping phenotype by using a novel combination of emibetuzumab and merestinib, or a pharmaceutically acceptable salt thereof, as part of a specific treatment regimen that provides unexpected beneficial therapeutic effects from the combined activity of these therapeutic agents in some cancer patients as compared to the therapeutic effects provided by either agent alone. Preferably, the cancer is lung, neuroblastoma, gastric, or colon cancer. More preferably, the cancer is gastric or lung cancer. Even more preferably, the cancer is lung cancer. Most preferably, the cancer is non-small cell lung cancer.

Accordingly, the present invention provides treatment of cancer with tumors bearing MET exon 14 skipping mutation(s) or MET exon 14 skipping phenotype in a patient, comprising administering to a cancer patient in need of such treatment an effective amount of emibetuzumab in combination with an effective amount of merestinib, or a pharmaceutically acceptable salt thereof. Preferably, the cancer in the aforementioned methods is lung, neuroblastoma, gastric, or colon cancer. More preferably, the cancer is gastric or lung cancer. Even more preferably, the cancer is carcinoma of the gastroesophageal junction. Even more preferably, the cancer is lung cancer. Most preferably, the cancer in the aforementioned methods is non-small cell lung cancer.

The invention further provides a pharmaceutical composition comprising emibetuzumab with one or more pharmaceutically acceptable carriers, diluents, or excipients, in combination with a pharmaceutical composition of merestinib with one or more pharmaceutically acceptable carriers, diluents, or excipients.

The invention also provides a kit comprising emibetuzumab and merestinib, or a pharmaceutically acceptable salt thereof.

The invention also provides a kit comprising a pharmaceutical composition comprising emibetuzumab, with one or more pharmaceutically acceptable carriers, diluents, or excipients, and a pharmaceutical composition comprising merestinib, or a pharmaceutically acceptable salt thereof, with one or more pharmaceutically acceptable carriers, diluents, or excipients.

The invention also provides a kit comprising an effective amount of emibetuzumab in combination with an effective amount of merestinib, or a pharmaceutically acceptable salt thereof, for simultaneous, separate or sequential use in the treatment of cancer with tumors bearing MET exon 14 skipping or MET exon 14 skipping phenotype. In a further embodiment of the invention, the cancer is lung, neuroblastoma, gastric, or colon cancer. More preferably, the cancer is gastric or lung cancer. Even more preferably, the cancer is carcinoma of the gastroesophageal junction. Even more preferably, the cancer is lung cancer. Most preferably, the cancer in the aforementioned methods is non-small cell lung cancer.

The invention further provides a combination comprising emibetuzumab and merestinib, or a pharmaceutically acceptable salt thereof, for simultaneous, separate or sequential use in the treatment of cancer with tumors bearing MET exon 14 skipping mutation(s) or MET exon 14 skipping phenotype. In a further embodiment of the invention, the cancer is lung, neuroblastoma, gastric, or colon cancer. More preferably, the cancer is gastric or lung cancer. Even more preferably, the cancer is carcinoma of the gastroesophageal junction. Even more preferably, the cancer is lung cancer. Most preferably, the cancer in the aforementioned methods is non-small cell lung cancer.

The invention further provides the use of a combination comprising emibetuzumab and meresitnib, or a pharmaceutically acceptable salt thereof, for use in therapy. A further embodiment of the invention is the use of the combination comprising emibetuzumab and merestinib, or a pharmaceutically acceptable salt thereof, for treating cancer with tumors bearing MET exon 14 skipping mutation(s) or MET exon 14 skipping phenotype. In a further embodiment of the invention, the cancer is lung, neuroblastoma, gastric, or colon cancer. More preferably, the cancer is gastric or lung cancer. Even more preferably, the cancer is carcinoma of the gastroesophageal junction. Even more preferably, the cancer is lung cancer. Most preferably, the cancer in the aforementioned methods is non-small cell lung cancer.

Another aspect of the present invention is treating cancer with tumors bearing MET exon 14 skipping mutation(s) or MET exon 14 skipping phenotype in a patient, comprising administering to a patient in need of such treatment wherein merestinib, or a pharmaceutically acceptable salt thereof, is administered at a dose between about 60 mg to about 160 mg daily on a 28-day cycle and emibetuzumab is administered at a dose between about 500 mg to about 2500 mg on about day 1 and about day 15 of the 28-day cycle of merestinib dosing. More preferably, merestinib, or a pharmaceutically acceptable salt thereof, is administered at a dose between about 80 mg to about 120 mg daily of a 28-day cycle and emibetuzumab is administered at a dose of about 750 mg on about day 1 and about day 15 of the 28-day cycle of the merestinib dosing. In yet a further embodiment, merestinib is administered orally and emibetuzumab is administered intravenously.

Each of the embodiments described herein envisions within its scope pharmaceutically acceptable salts of the compounds referenced or described herein. Accordingly, the phrase "or a pharmaceutically acceptable salt thereof' is implicit in the references to or descriptions of all compounds herein.

The phrase "MET exon 14 skipping mutation", "exon 14 skipping mutation", "MET exon 14 skipping", "exon 14 skipping", or grammatical versions thereof, as used herein, refer to somatic mutations in the gene for MET, which, upon translation of the mRNA transcripts expressed thereby, result in cellular expression of MET polypeptides wherein exon 14 is largely or entirely deleted.

The phrases "MET exon 14 skipping phenotype", "exon 14 skipping phenotype", or grammatical versions thereof, as used herein refer to any single somatic point mutation in the gene for MET which, upon translation of the mRNA transcripts expressed thereby, result in cellular expression of MET polypeptides mutated at Y1003, D1002 or R1004 and which have a diminished ability to bind the ubiquitin ligase CBL, resulting in a MET protein with increased stability and oncogenic potential.

The term "K*_{D}*", as used herein, is intended to refer to the equilibrium dissociation constant of a particular antibody-antigen or antibody fragment-antigen interaction.

The phrase "specifically binds" as used herein in reference to the affinity of aantibody, or antigen-binding fragment thereof, for the MET-ECD is intended to mean, unless indicated otherwise, a K*_{D}* of less than about 1 x 10⁻⁸ M, preferably, less than about 1 x 10⁻⁹ M as determined by common methods known in the art, including by use of a surface plasmon resonance (SPR) biosensor at 25°C (for MET-ECD).

As used herein, the term "C8-H241 antibody" or " C8-H241 Ab" refers to an antibody comprising: a LCVR whose amino acid sequence is that given in SEQ ID NO: 1, and a HCVR whose amino acid sequence is that given in SEQ ID NO: 3, wherein that C8-H241 Ab specifically binds to MET-ECD. In some embodiments, a C8-H241 Ab is a C8-H241-IgG4 Ab, an antibody comprising: a LC whose amino acid sequence is that given in SEQ ID NO: 5, and a HC whose amino acid sequence is that given in SEQ ID NO: 7 and that specifically binds to MET-ECD.

As used herein, the term "emibetuzumab" refers to a C8-H241-IgG4 antibody comprising: two light chains, each of whose amino acid sequence is that given in SEQ ID NO: 5, and two heavy chains, each of whose amino acid sequence is that given in SEQ ID NO: 7 and that specifically binds to MET-ECD (see, WHO Drug Information, Proposed International Nonproprietary Names (INN) List 111, Volume 28, No. 2, July 2014).

In certain embodiments, a C8-H241 Ab or a C8-H241-IgG4 Ab, including, but not limited to, emibetuzumab binds MET-ECD with a K*_{D}* value of between about 100 nM to about 1 pM, preferably, between about 10 nM to about 10 pM, more preferably, between about 10 nM to about 100 pM, more preferably, between about 2.5 nM to about .5 nM, and most preferably about 1 nM, as determined by a surface plasmon resonance (SPR) based assay (such as the BIAcore assay as described in PCT Application Publication No. WO 2005/012359) conducted at 25 °C. Antibody affinities may also be determined by ELISA; and competition assays (e.g., a RIA), for example.

The terms "MET polypeptide", "MET receptor", "MET", "HGF receptor" or "HGFR" are used interchangeably herein and, unless otherwise indicated, are intended to refer to the human receptor tyrosine kinase, as well as functionally active, mutated forms thereof, that bind human hepatocyte growth factor. Specific examples of MET include, e.g., a human polypeptide encoded by the nucleotide sequence provided in GenBank accession no. NM_000245, or the human protein encoded by the polypeptide sequence provided in GenBank accession no. NP_000236.

The extracellular domain of MET has the amino acid sequence shown in, for example, SEQ ID NO: 9. However, amino acids 1-24 of SEQ ID NO: 9 comprise the signal sequence. Therefore, unless stated otherwise, the term "MET-ECD" as used herein means the protein beginning and ending at amino acids 25 and 932, respectively, of SEQ ID NO: 9 (*i.e.,* SEQ ID NO: 10). The SEMA domain consists of approximately 500 amino acid residues at the N-terminus of MET, and contains the α-chain (amino acid residues 25-307 of SEQ ID NO: 9 (*i.e.,* SEQ ID NO: 11) and part of the β-chain (amino acid residues 308-519 of SEQ ID NO: 18 (*i.e.,* SEQ ID NO: 12)).

Unless indicated otherwise, the term "antibody" refers to an immunoglobulin molecule comprising two heavy chains and two light chains interconnected by disulfide bonds. The amino terminal portion of each chain includes a variable region of about 100 to about 110 amino acids primarily responsible for antigen recognition via the complementarity determining regions (CDRs) contained therein. The carboxy-terminal portion of each chain defines a constant region primarily responsible for effector function.

As used herein, the term "antigen-binding fragment" refers to any antibody fragment that retains the ability to bind to its antigen. Such "antigen-binding fragments" can be selected from the group consisting of Fv, scFv, Fab, F(ab')₂, Fab', scFv-Fc fragments and diabodies. An antigen-binding fragment of an antibody will typically comprise at least one variable region. Preferably, an antigen-binding fragment comprises a heavy chain variable region (HCVR) and a light chain variable region (LCVR). More preferably, an antigen-binding fragment as used herein comprises a HCVR and a LCVR which confers antigen-binding specificity to MET-ECD (i.e., a "MET-ECD binding fragment").

As used herein, the term "light chain variable region (LCVR)" refers to a portion of a LC of an antibody molecule that includes amino acid sequences of Complementarity Determining Regions (CDRs; i.e., LCDR1, LCDR2, and LCDR3), and Framework Regions (FRs).

As used herein, the term "heavy chain variable region (HCVR)" refers to a portion of a HC of an antibody molecule that includes amino acid sequences of Complementarity Determining Regions (CDRs; i.e., HCDR1, HCDR2, and HCDR3), and Framework Regions (FRs).

As used herein, the terms "complementarity determining region" and "CDR", refer to the non-contiguous antigen combining sites found within the variable region of LC and HC polypeptides of an antibody or an antigen-binding fragment thereof. These particular regions have been described by others including Kabat, et al., Ann. NY Acad. Sci. 190:382-93 (1971); Kabat et al., J. Biol. Chem. 252:6609-6616 (1977); Kabat, et al., Sequences of Proteins of Immunological Interest, Fifth Edition, U.S. Department of Health and Human Services, NIH Publication No. 91-3242 (1991); Chothia, et al., J. Mol. Biol. 196:901-917 (1987); MacCallum, et al., J. Mol. Biol., 262:732-745 (1996); and North, et al., J. Mol. Biol., 406, 228-256 (2011), where the definitions include overlapping or subsets of amino acid residues when compared against each other.

The CDRs are interspersed with regions that are more conserved, termed framework regions ("FR"). Each LCVR and HCVR is composed of three CDRs and four FRs, arranged from amino-terminus to carboxy-terminus in the following order: FR1, CDR1, FR2, CDR2, FR3, CDR3, FR4. The three CDRs of the light chain are referred to as "LCDR1, LCDR2, and LCDR3" and the three CDRs of the HC are referred to as "HCDR1, HCDR2, and HCDR3." The CDRs contain most of the residues which form specific interactions with the antigen. The numbering and positioning of CDR amino acid residues within the LCVR and HCVR regions is in accordance with known conventions (e.g., Kabat (1991), Chothia (1987), and/or North (2011)). In different embodiments of the invention, the FRs of the antibody may be identical to the human germline sequences, or may be naturally or artificially modified.

In certain embodiments, C8-H241 antibodies, including C8-H241-IgG4 antibodies or emibetuzumab, for the uses of the present invention may be altered to increase or decrease the extent to which the antibody is glycosylated. Addition or deletion of glycosylation sites to an antibody may be conveniently accomplished by altering the amino acid sequence such that one or more glycosylation sites is created or removed.

Where a C8-H241 antibody, including, but not limited to, emibetuzumab, comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region (see, e.g., Wright et al. TIBTECH 15:26-32 (1997)). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In some embodiments, the C8-H241 antibody, including, but not limited to, emibetuzumab may have a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycosyl structures attached to Asn297 (e.g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function (see, US Patent Publication Nos. US 2003/0157108 and US 2004/0093621, for example). Examples of publications related to "defucosylated" or "fucose-deficient" antibody variants include: US 2003/0157108; WO 2000/61739; WO 2001/29246; US 2003/0115614; US 2002/0164328; and Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1; and WO 2004/056312 A1), and knockout cell lines, such as alpha-1,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO 2003/085107).

Unless indicated otherwise, when referring to an amino acid residue in an antibody by a number, the EU numbering system is used herein as it is conventionally used in the art (see, Kabat, et al., Sequences of Proteins of Immunological Interest, Fifth Edition, U.S. Department of Health and Human Services, NIH Publication No. 91-3242 (1991), for example).

As used herein, the term "kit" refers to a package comprising at least two separate containers, wherein a first container contains a C8-H241 antibody, preferably, emibetuzumab, and a second container contains an anti-MET inhibitor. As used herein, the term "kit" also refers to a package comprising at least two separate containers, wherein a first container contains emibetuzumab, and a second container contains merestinib, or a pharmaceutically acceptable salt thereof. A "kit" may also include instructions to administer all or a portion of the contents of these first and second containers to a cancer patient, preferably, a gastric cancer patient, a patient having carcinoma of the GEJ, a lung cancer patient, or a NSCLC patient and preferably a cancer patient with tumors with MET exon 14 skipping mutation(s) or MET exon 14 skipping phenotype.

As used herein, the terms "treating," "to treat," or "treatment" refers to restraining, slowing, stopping, reducing, or reversing the progression or severity of an existing symptom, disorder, condition, or disease.

As used herein, the term "patient" refers to a mammal, preferably a human.

As used herein, the terms "cancer" and "cancerous" refer to or describe the physiological condition in patients that is typically characterized by unregulated cell proliferation. Included in this definition are benign and malignant cancers. By "early stage cancer" or "early stage tumor" is meant a cancer that is not advanced or metastatic or is classified as a Stage 0, I, or II cancer. Examples of cancer include, but are not limited to, gastric cancer, preferably, carcinoma of the gastroesophageal junction, and lung cancer, preferably NSCLC.

In some embodiments of the present invention, the cancer patients are selected for treatment with a combination therapy disclosed herein on the basis of having a tumor in which MET is expressed or overexpressed. Preferably, the MET expression status of a cancer patient's tumor is determined by using an immunohistochemistry (IHC) assay, PCR assay, gene sequencing assay and/or fluorescence in-situ hybridization (FISH) assay suitable for the detection of MET or MET gene amplification. More preferably, an IHC method for determining whether a cancer patient's tumor expresses or overexpresses MET is performed essentially as described in Example 7 of PCT International Publication WO 2013/169532 using an anti-MET antibody, or an antigen-binding fragment thereof, disclosed therein that specifically binds to MET-ECD, wherein said anti-MET antibody comprises a LC and a HC, wherein the amino acid sequence of the LC and HC is that given in SEQ ID NO: 13 and SEQ ID NO: 14, respectively. In some embodiments, a patient is selected for treatment with the combination therapies of the present invention after a sample of a cancer patient's tumor has been determined expressed or overexpress MET by use of an IHC assay wherein the assay comprises the step of contacting the sample with a MET antibody, or antigen-binding fragment thereof, wherein the antibody, or fragment thereof, comprises a LC and a HC, wherein the amino acid sequence of the LC and HC is that given in SEQ ID NO: 13 and SEQ ID NO: 14, respectively. In various embodiments of the methods of the present invention, the aforementioned IHC assay of the patient's tumor is performed using a formalin-fixed and paraffin-embedded sample of the patient's tumor.

In some embodiments of the present invention, the cancer patients are selected for treatment with a combination therapy disclosed herein on the basis of having a tumor with MET exon 14 skipping mutations or MET exon 14 phenotype. Preferably, the MET exon 14 skipping mutation status of a cancer patient's tumor is determined by next generation gene sequencing methodologies. More preferably, the MET exon 14 skipping mutations or MET exon 14 phenotype of a cancer patient's tumor is determined by using Hybridization-captured Next Generation Sequencing (see., *e.g.,* Schrock, A. B., et al., J Thoracic Oncology 2016, 9(11): 1493-1502). More preferably, the MET exon 14 phenotype of a cancer patient's tumor is determined by using the nCounter Analysis System (NANOSTRING® Technologies), a fluorescence-based platform for multiplexed digital mRNA profiling without amplification or generation of cDNA (see., *e.g.,* Geiss, G. K., et al, Nature Biotechnology 2008, 26: 317-325).

A main advantage of the combination treatments of the invention is the ability of producing marked anti-cancer effects in a patient without causing significant toxicities or adverse events, so that the patient benefits from the combination treatment method overall. The efficacy of the combination treatment of the invention can be measured by various endpoints commonly used in evaluating cancer treatments, including but not limited to, tumor regression, tumor weight or size shrinkage, time to progression, overall survival, progression free survival, overall response rate, duration of response, and quality of life. The therapeutic agents used in the invention may cause inhibition of metastatic spread without shrinkage of the primary tumor, may induce shrinkage of the primary tumor, or may simply exert a tumoristatic effect. Because the invention relates to the use of a combination of unique anti-tumor agents, novel approaches to determining efficacy of any particular combination therapy of the present invention can be optionally employed, including, for example, measurement of plasma or urinary markers of angiogenesis and measurement of response through radiological imaging.

As used herein, the term "effective amount" refers to the amount or dose of a C8-H241 antibody, emibetuzumab, and/or to the amount or dose of an anti-MET inhibitor, merestinib, which, upon single or multiple dose administration to the patient, optionally in combination with one or more other active agents, as the case may be, provides an effective response in the patient under diagnosis or treatment. As used herein, the term "effective amount" also refers to the amount or dose of a C8-H241 antibody, emibetuzumab, and to the amount or dose of merestinib, or a pharmaceutically acceptable salt thereof, which, upon single or multiple dose administration to the patient, optionally, in combination with one or more other active agents, provides an effective response in the patient under diagnosis or treatment. It is understood that a combination therapy of the present invention is carried out by administering a C8-H241 antibody, emibetuzumab, together with an anti-MET inhibitor in any manner which provides effective levels of the C8-H241 antibody, emibetuzumab, and the anti-MET inhibitor, merestinib, in the body. It is also understood that a combination therapy of the present invention is carried out by administering emibetuzumab together with merestinib, or a pharmaceutically acceptable salt thereof, in any manner which provides effective levels of emibetuzumab and merestinib in the body.

As used herein, the terms "effective response" of a patient or a patient's "responsiveness" to treatment with a combination of agents, or "therapeutic effect" refers to the clinical or therapeutic benefit(s) imparted to a patient upon administration of emibetuzumab and merestinib, or a pharmaceutically acceptable salt thereof. Such benefit(s) include any one or more of: extending survival (including overall survival and progression free survival); resulting in an objective response (including a complete response or a partial response); tumor regression, tumor weight or size shrinkage, longer time to disease progression, increased duration of survival, longer progression free survival, improved overall response rate, increased duration of response, and improved quality of life and/or improving signs or symptoms of cancer, etc.

An effective amount can be readily determined by the attending diagnostician, as one skilled in the art, by the use of known techniques and by observing results obtained under analogous circumstances. In determining the effective amount for a patient, a number of factors are considered by the attending diagnostician, including, but not limited to: the species of patient; its size, age, and general health; the specific disease or disorder involved; the degree of or involvement or the severity of the disease or disorder; the response of the individual patient; the particular compound administered; the mode of administration; the bioavailability characteristics of the preparation administered; the dose regimen selected; the use of concomitant medication; and other relevant circumstances.

Emibetuzumab is generally effective over a wide dosage range in the combination of the present invention. For example, dosages normally are given on days one and fifteen of a 28-day treatment cycle and each dose falls within the range of about 500 mg to about 2500 mg, preferably about 750 mg to about 2000 mg, and most preferably about 750 mg. In addition, merestinib, or a pharmaceutically acceptable salt thereof, is generally effective over a wide dosage range in the combination of the present invention. For example, dosages per 28-day cycle normally fall within the range of daily dosing of about 60 to 160 mg, preferably about 80 to about 120 mg, and most preferably 120 mg. In some instances dosage levels below the lower limit of the aforesaid ranges for emibetuzumab and merestinib, or a pharmaceutically acceptable salt thereof, may be more than adequate, while in other cases smaller or still larger doses may be employed with acceptable side effects, and therefore the above dosage range is not intended to limit the scope of the invention in any way. When given in combination with an anti-MET inhibitor, for example, over a 28-day cycle, emibetuzumab, is administered on days one and fifteen within the range of about 500 mg to about 2500 mg and merestinib, or a pharmaceutically acceptable salt thereof, is administered daily within the range of about 60 to 120 mg. When given in combination, for example, over a 28-day cycle, emibetuzumab is administered on day one and day fifteen within the range of about 750 mg to about 2000 mg and merestinib, or a pharmaceutically acceptable salt thereof, is administered on day one and day fifteen within the range of about 80-120 mg. Optionally, a 21-day cycle may be employed with dose given daily the dosage of merestinib, or a pharmaceutically acceptable salt thereof, within the range of about 60-120 mg, more preferably, 80-120 mg and the dosage of emibetuzumab in the range of about 750 mg to about 200 mg, more preferably, 1000 mg given on day one and day 21.

Emibetuzumab, and merestinib, or a pharmaceutically acceptable salt thereof, are preferably formulated as pharmaceutical compositions administered by any route which makes the compound bioavailable. The route of administration may be varied in any way, limited by the physical properties of the drugs and the convenience of the patient and the caregiver. Preferably, merestinib, or a pharmaceutically acceptable salt thereof, compositions are for oral administration, although parenteral administration, including intravenous or subcutaneous administration, may be used. (See, *e.g*., Remington: The Science and Practice of Pharmacy (D.B. Troy, Editor, 21st Edition, Lippincott, Williams & Wilkins, 2006.) Pharmaceutical compositions of emibetuzumab are for parenteral administration, such as intravenous or subcutaneous administration. More preferably, emibetuzumab compositions comprise about 10 to about 20 mg/ml of emibetuzumab, about 10 to about 20 mM histidine buffer, pH 5.5-6.0, about 75 mM to about 150 mM sodium chloride, about 0.01% to about 0.06% polysorbate 80, and, optionally, about 100 mM to about 150 mM glycine. Preferably, emibetuzumab compositions are formulated for intravenous administration and comprise about 20 mg/ml of emibetuzumab, about 10 mM histidine buffer, pH 5.5, about 150 mM sodium chloride, and about 0.06% polysorbate 80. Such pharmaceutical compositions and processes for preparing same are well known in the art. (See, *e.g*., Remington: The Science and Practice of Pharmacy (D.B. Troy, Editor, 21st Edition, Lippincott, Williams & Wilkins, 2006.)

As used herein, the term "in combination with" refers to the administration of merestinib, or a pharmaceutically acceptable salt thereof, and emibetuzumab either separately, simultaneously, or sequentially in any order, such as for example, at repeated intervals as during a standard course of treatment for a single cycle or more than one cycle, such that one agent can be administered prior to, at the same time, or subsequent to the administration of the other agent, or any combination thereof. It can also mean that one drug is given alone first until patients progress before the second drug is being added on.

A main advantage of the combination treatments of the invention is the ability of producing marked anti-cancer effects in a patient without causing significant toxicities or adverse events, so that the patient benefits from the combination treatment method overall. The efficacy of the combination treatment of the invention can be measured by various endpoints commonly used in evaluating cancer treatments, including but not limited to, tumor regression, tumor weight or size shrinkage, time to progression, overall survival, progression free survival, overall response rate, duration of response, and quality of life. The therapeutic agents used in the invention may cause inhibition of metastatic spread without shrinkage of the primary tumor, may induce shrinkage of the primary tumor, or may simply exert a tumoristatic effect. Because the invention relates to the use of a combination of unique anti-tumor agents, novel approaches to determining efficacy of any particular combination therapy of the present invention can be optionally employed, including, for example, measurement of plasma or urinary markers of angiogenesis and/or cell cycle activity, tissue-based biomarkers for angiogenesis and/or cell cycle activity and/or circulating levels of MET exon 14 skipping polypeptides and/or MET mutations leading to MET exon 14 skipping or exon 14 skipping phenotye, and measurement of response through radiological imaging.

The following examples illustrate the unexpected benefit of the present combinations.

### Example 1

### Evaluation of the combination of merestinib (LY2801653) and emibetuzumab (LY2875358) in a xenograft tumor model bearing MET exon 14 skipping mutation(s)

To determine the efficacy of merestinib, in combination with emibetuzumab, in an Hs746t-derived xenograft mouse model of human gastric carcinoma and to compare the combination effect to that of either monotherapy, studies conducted essentially as described below may be performed. Hs746t is a gastric cancer cell line known to have MET exon 14 skipping mutation(s) and MET amplification (Asaoka et al., Biochem Biophys Res Commun 394:1042-1046).

### Study Designs and Methods:

Female athymic nude mice (Envigo) are used for this study. Food and water are available ad libitum. Animals are acclimated for 1 week prior to any experimental manipulation. The study is performed in accordance with AAALAC accredited institutional guidelines.

Merestinib is formulated as a solution in 10% PEG 400/90% (20% Captisol in water). Emibetuzumab is diluted in phosphate buffered saline (PBS). Solutions are freshly prepared every 7 days.

Obtain Hs746t cells from ATCC (American Type Culture Collection) and maintain in RPMI 1640 with 10% fetal bovine serum at 37 °C in 5% CO₂. Expand cells in culture, harvest, and wash in Hank's Balanced Salt Solution (HBSS, GIBCO®). Implant sub-confluent MKN45 cells into 60 female *nu*/*nu* mice at a concentration of 1 x 10⁷ cells in 0.2 ml HBSS subcutaneously into the hind flank of the animal. Randomize the animals when tumors reach an average volume of 350 mm³ by tumor volume into four treatment groups (*n* = 7): PBS vehicle control; merestinib (at 6 mg/kg or 12 mg/kg), qd orally; emibetuzumab, at 10 mg/kg, IP, qw; merestinib (at 6 mg/kg or 12 mg/kg) qd orally and emibetuzumab at 10 mg/kg, IP, qw.

Administer merestinib by oral gavage at either 6 mg/kg or 12 mg/kg in a dose volume of 0.2 mL in 10% polyethylene glycol 400/ 90% of a mixture of 20% Captisol in water, and antibody treatments by intraperitoneal injection (IP) starting on day 12 after tumor cell implantation when average tumor volume of 350 mm³ and ending on Day 33. Inject animals IP in a dosing volume of 10 mg/kg. After 14 days of dosing (Study Day 27), the animals in the control group are sacrificed because of tumor volumes exceeding >2000 mm³. After the 28-day dosing period, animals in the single agent arms are sacrificed. Tumors in the animals in the combination treatment groups are observed for an additional 2 weeks.

In both studies, animals are sacrificed using CO₂ and cervical dislocation when tumors grow larger than 2000 mm³, or at the end of study.

Dose animals in treatment group 4, with merestinib first, followed by emibetuzumab, 30 - 45 minutes later. Record tumor growth and body weight changes at least twice a week. Body weight measurement during the course of the study, is a general indicator of tolerability.

Measure tumor growth with calipers and record body weights twice weekly. Calculate tumor volumes by the formula *Volume (mm³)* = *L x W² (π*/*6)* where *L* represents the larger diameter and *W* the smaller diameter. Calculate T/C% using the formula *T*/*C%* = *100 x* Δ*T*/Δ*C.* Where Δ*T* = mean tumor volume of the drug-treated group on the final day of the study - mean tumor volume of the drug-treated group on the initial day of the dosing and Δ*C* = mean tumor volume of the control group on the final day of the study - mean tumor volume of the control group on the initial day of the dosing. Calculate changes in body weight by the formula *(Weight on observation day* - *Weight on day 12)* / *Weight on Day 12 x 100.* Calculate test for significant differences between treatment groups by *RM ANOVA* using the JMP (v.9.0.3) statistical package (SAS Institute Inc., Cary, NC, USA).

### Results:

Merestinib inhibits Hs746t cell proliferation with IC₅₀=34nM and totally eliminates pMET at 65-100nM. Emibetuzumab slightly inhibits Hs746t cell proliferation (IC₅₀>100nM), reduces 10-20% cell surface MET, and shows no effect on pMET expression (at 130-650nM).

In the Hs746t xenograft model, merestinib (12 mg/kg) treatment results in 91.8% tumor regression after 21 day dosing, while 6 mg/kg merestinib provides transient tumor regression followed by re-growth while on treatment with T/C=18.3% after 21 day dosing. No tumor regrowth is observed in 6/7 mice in the 12mg/kg merestinib cohort during the 5 weeks post-treatment. Emibetuzumab treatment provides transient tumor regression (37.7%) after 3 doses, but tumors are observed to regrow while on treatment. Combination of 6 mg/kg merestinib and 10 mg/kg emibetuzumab results in 85% tumor regression for the duration of the 28 day dosing period and the treatment is well tolerated. Regrowth of tumors in animals is observed upon termination of this combination treatment.

All animals are shown to have survived the dosing and observation period. No loss in body weight is observed in the combination treatment group. All mice in the vehicle control group display disease progression.

To assess the effect of the combination of merestinib and emibetuzumab, a repeated measures ANOVA model is fit on log transformed tumor volume using the mixed procedure in SAS software (version 9.3, Cary, NC) followed by a 2x2 interaction test to test for statistical significance of the combination of the two single agents. The observed percent reduction of the combination on day 28 is 83.1%. The expected percent reduction of the combination on day 28 is 81.5%. The p-value from the 2x2 interaction test on day 28 is not significant at p=0.654. All pairwise comparisons between the combination group and each single agent group on day 33 are also statistically significant. By inspection of the means, these results indicate that the combination group is statistically smaller than each single agent group. Full statistical analysis is not feasible as the animals in the vehicle control groups were removed after 14 days of dosing as the animals are sacrificed due to tumor burden.

The data of Example 1 indicates that the combination of merestinib and emibetuzumab results in tumor regression.

### Example 2

### Evaluation of sequential and concurrent administration of merestinib (LY2801653) and emibetuzumab (LY2875358) in a xenograft tumor model bearing MET exon 14 skipping mutation(s)

To determine the efficacy of low-dose merestinib, in sequential and concurrent combination with emibetuzumab, in an Hs746t-derived xenograft mouse model of human gastric carcinoma, and to compare the combination effect to that of higher-dose emibetuzumab monotherapy, studies conducted essentially as described below may be performed.

### Study Designs and Methods:

Female athymic nude mice (Envigo) are used for this study. Food and water are available ad libitum. Animals are acclimated for 1 week prior to any experimental manipulation. The study is performed in accordance with AAALAC accredited institutional guidelines.

Merestinib is formulated as a solution in 10% PEG 400/90% (20% Captisol in water). Emibetuzumab and IgG control are diluted in PBS. Solutions are freshly prepared every 7 days.

Obtain, maintain, expand, harvest, and wash Hs746t cell as in Example 1. Implant cells into 36 female *nu*/*nu* mice at a concentration of 2 x 10⁶ cells in 0.2 ml HBSS subcutaneously into the hind flank of the animal. Randomize the animals (N=36) when tumors reach an average volume of 350 mm³ by tumor volume into five treatment groups (*n* = 6): combined vehicle control of 10% PEG 400/90% (20% Captisol in water) administered by oral gavage qd, and IgG control at 10 mg/kg administered via IP injection qw; emibetuzumab (10 mg/kg and 20 mg/kg) administered via IP injections qw for 4 cycles; emibetuzumab (10 mg/kg) administered via IP qw for 7 cycles with concurrent administration of merestinib (6 mg/kg) administered orally qd for 50 days; and merestininb (6mg/kg), administered orally qd for 17 days, as single agent, with sequential combination administration of emibetuzumab (10 mg/kg) administered via IP qw for 6 cycles starting on day 37 (whereupon tumor regression and regrowth to 500 mm³ after single agent merestinib (day 37) is observed), for a total of 58 days of merestinib dosing. After the 28-day dosing period, animals in the single agent arms are sacrificed. Tumors in the animals in the combination treatment groups are monitored for continued growth or regrowth of tumors.

In these studies, animals are sacrificed using CO₂ and cervical dislocation when tumors grow larger than 2000 mm³, or at the end of study.

Measure tumor volumes and body weight at least twice weeky. Body weight measurement during the course of the study, is a general indicator of tolerability.

Measure tumor growth with calipers and record body weights twice weekly. Calculate tumor volumes by the formula *Volume (mm³) = L x W² (π*/*6)* where *L* represents the larger diameter and *W* the smaller diameter. Calculate T/C% using the formula *T*/*C%* = *100 x* Δ*T*/Δ*C.* Where Δ*T* = mean tumor volume of the drug-treated group on the final day of the study - mean tumor volume of the drug-treated group on the initial day of the dosing and Δ*C* = mean tumor volume of the control group on the final day of the study - mean tumor volume of the control group on the initial day of the dosing. Calculate changes in body weight by the formula *(Weight on observation day* - *Weight on day 12)* / *Weight on Day 12 x 100.* Calculate test for significant differences between treatment groups by *RM ANOVA* using the JMP (v.9.0.3) statistical package (SAS Institute Inc., Cary, NC, USA).

Transform tumor volume to the log scale to equalize variance across time and treatment groups. Analyze the log volume data with a two-way repeated measures analysis of variance by time and treatment using the MIXED procedures in SAS software (Version 9.3). The correlation model for the repeated measures is spatial power. Compare treated groups to the control group at each time point. The MIXED procedure is also used separately for each treatment group to calculate adjusted means and standard errors at each time point. Both analyses account for the autocorrelation within each animal and the loss of data that occurs when animals are removed or lost before the end of the study. The adjusted means and standard errors are plotted for each treatment group versus time. Full statistical analysis is not feasible as the animals in the vehicle control groups were removed after 17 days of dosing (study day 37), as half the animals from the vehicle group are removed and sacrificed due to tumor burden (tumor volume >2000 mm³).

### Results:

In the Hs746t xenograft model, merestinib (6 mg/kg qd for 58 days) results in transient tumor regression, with maximal regression (30.2%) occurring on Day 27 (7 days of dosing), followed by re-growth while on treatment; by Day 37, tumor volumes approach about 500 mm³. Sequential addition of emibetuzumab (10 mg/kg qw for 6 cycles) to merestinib treatment on Day 37 results in tumor regression in 5/6 animals, with complete regression and no tumor regrowth over a 26 day observation period after dosing completion in 2/6 animals. Transient tumor regression with slow regrowth during post-treatment observation is evident in an additional 2/6 animals. In an additional animal, tumor regression occurs to about 50 mm³ with slow regrowth during the post-treatment observation. In the one non-responding animal, tumor size on day 37 is notably the largest (approximately 1200 mm³) in the entire cohort, when emibetuzumab treatment is initiated.

Concurrent treatment with merestinib (6 mg/kg qd for 50 days) and emibetuzumab (10 mg/kg qw for 7 cycles) results in initial tumor regression (87.5% on day 37), but regression proved transient, with slow tumor regrowth over the course of treatment. Tumor size approaches pre-treatment volumes by Day 70, and additional monitoring for a week post-treatment results in tumor size doubling in volume in 4/6 animals.

Single-agent emibetuzumab treatment (10 mg/kg and 20 mg/kg qw 28 days) results in transient tumor regression at both doses. In the 10 mg/kg cohort, maximum regression (16.0%) is observed on Day 27, with maximum regression (58.9%) in the 20 mg/kg cohort occurring on Day 37. Tumor volumes from both cohorts were not statistically significant (p = 0.129).

In these studies, body weight measurements provide an indication of the tolerability of the various treatments. There were no significant decreases in body weight observed in any group.

The data of Example 2 indicates that the concurrent combination of low-dose merestinib and emibetuzumab results in transient tumor regression, with sequential combination of emibetuzumab to low-dose merestinib resulting in longer duration of treatment response.

## Claims

1. A combination comprising emibetuzumab and merestinib, or a pharmaceutically acceptable salt thereof, for simultaneous, separate or sequential use in therapy.

2. A combination comprising emibetuzumab and meristinib, or a pharmaceutically acceptable salt thereof, for simultaneous, separate or sequential use in the treatment of cancer with tumors bearing MET exon 14 skipping or MET exon 14 skipping phenotype.

3. The combination for use according to Claim 1 or 2 wherein merestinib, or a pharmaceutically acceptable salt thereof, is to be administered at a dose of between about 60 mg to about 160 mg daily on a 28-day cycle and emibetuzumab is to be administered at a dose of about 500 mg to about 2000 mg on about day 1 and about day 15 of the 28-day cycle of merestinib dosing

4. The combination for use according to Claim 3 wherein merestinib, or a pharmaceutically acceptable salt thereof, is to be administered at a dose of between about 80 mg to about 120 mg daily on a 28-day cycle and emibetuzumab is to be administered at a dose of about 750 mg on about day 1 and about day 15 of the 28-day cycle of merestinib dosing.

5. The combination for use according to Claims 3 or 4 wherein merestinib, or a pharmaceutically acceptable salt thereof, is to be
administered orally and emibetuzumab is to be administered intravenously.

6. The combination for use according to Claim 2-5 wherein the cancer is gastric cancer or lung cancer.

7. The combination for use according to Claims 2-6 wherein the cancer is lung cancer.

8. The combination for use according to Claim 7 wherein the cancer is non-small cell lung cancer.

9. A kit comprising an effective amount of emibetuzumab in combination with an effective amount of the compound merestinib, or a pharmaceutically acceptable salt thereof, for simultaneous, separate, or sequential use in the treatment of cancer with tumors bearing MET exon 14 skipping or MET exon 14 skipping phenotype.

10. The kit for use according to Claim 9 wherein the cancer is gastric cancer or lung cancer.

11. The kit for use according to Claims 9 or 10 wherein the cancer is lung cancer.

12. The kit for use according to Claim 11 wherein the cancer is non-small cell lung cancer.

## Patentansprüche

1. Kombination, umfassend Emibetuzumab und Merestinib oder ein pharmazeutisch annehmbares Salz davon, zur gleichzeitigen, separaten oder aufeinanderfolgenden Verwendung in der Therapie.

2. Kombination, umfassend Emibetuzumab und Merestinib oder ein pharmazeutisch annehmbares Salz davon, zur gleichzeitigen, separaten oder aufeinanderfolgenden Verwendung bei der Behandlung von Krebs mit Tumoren, die ein MET-Exon-14-Skipping oder einen MET-Exon-14-Skipping-Phänotyp tragen.

3. Kombination zur Verwendung nach Anspruch 1 oder 2, wobei Merestinib oder ein pharmazeutisch annehmbares Salz davon mit einer Dosis von zwischen etwa 60 mg und etwa 160 mg täglich in einem 28-Tage-Zyklus zu verabreichen ist und Emibetuzumab mit einer Dosis von zwischen etwa 500 mg und etwa 2000 mg etwa am Tag 1 und etwa am Tag 15 des 28-Tage-Zyklus der Merestinib-Dosierung zu verabreichen ist.

4. Kombination zur Verwendung nach Anspruch 3, wobei Merestinib oder ein pharmazeutisch annehmbares Salz davon mit einer Dosis von zwischen etwa 80 mg und etwa 120 mg täglich in einem 28-Tage-Zyklus zu verabreichen ist und Emibetuzumab mit einer Dosis von etwa 750 mg etwa am Tag 1 und etwa am Tag 15 des 28-Tage-Zyklus der Merestinib-Dosierung zu verabreichen ist.

5. Kombination zur Verwendung nach Anspruch 3 oder 4, wobei Merestinib oder ein pharmazeutisch annehmbares Salz davon oral zu verabreichen ist und Emibetuzumab intravenös zu verabreichen ist.

6. Kombination zur Verwendung nach Anspruch 2-5, wobei der Krebs Magenkrebs oder Lungenkrebs ist.

7. Kombination zur Verwendung nach Anspruch 2-6, wobei der Krebs Lungenkrebs ist.

8. Kombination zur Verwendung nach Anspruch 7, wobei der Krebs nicht-kleinzelliger Lungenkrebs ist.

9. Kit, umfassend eine wirksame Menge Emibetuzumab in Kombination mit einer wirksamen Menge der Verbindung Merestinib oder eines pharmazeutisch annehmbaren Salzes davon, zur gleichzeitigen, separaten oder aufeinanderfolgenden Verwendung bei der Behandlung von Krebs mit Tumoren, die ein MET-Exon-14-Skipping oder einen MET-Exon-14-Skipping-Phänotyp tragen.

10. Kit zur Verwendung nach Anspruch 9, wobei der Krebs Magenkrebs oder Lungenkrebs ist.

11. Kit zur Verwendung nach Anspruch 9 oder 10, wobei der Krebs Lungenkrebs ist.

12. Kit zur Verwendung nach Anspruch 11, wobei der Krebs nicht-kleinzelliger Lungenkrebs ist.

## Revendications

1. Combinaison comprenant emibetuzumab et merestinib, ou sel pharmaceutiquement acceptable de celle-ci, pour l'utilisation simultanée, séparée ou séquentielle en thérapie.

2. Combinaison comprenant emibetuzumab et merestinib, ou sel pharmaceutiquement acceptable de celle-ci, pour utilisation simultanée, séparée ou séquentielle dans le traitement de cancer avec des tumeurs portant une omission d'exon 14 de MET ou un phénotype à omission d'exon 14 de MET.

3. Combinaison pour l'utilisation selon la revendication 1 ou 2, dans laquelle merestinib, ou un sel pharmaceutiquement acceptable de celui-ci, est destiné à être administré à une dose d'entre environ 60 mg et environ 160 mg par jour durant un cycle de 28 jours et emibetuzumab est destiné à être administré à une dose d'environ 500 mg à environ 2000 mg, environ au jour 1 et environ au jour 15 du cycle de 28 jours de dosage de merestinib

4. Combinaison pour l'utilisation selon la revendication 3, dans laquelle merestinib, ou un sel pharmaceutiquement acceptable de celui-ci, est destiné à être administré à une dose d'entre environ 80 mg à environ 120 mg par jour durant un cycle de 28 jours et emibetuzumab est destiné à être administré à une dose d'environ 750 mg, environ au jour 1 et environ au jour 15 du cycle de 28 jours de dosage de merestinib.

5. Combinaison pour l'utilisation selon les revendications 3 ou 4, dans laquelle merestinib, ou un sel pharmaceutiquement acceptable de celle-ci, est destiné à être administré par voie orale et emibetuzumab est destiné à être administré par voie intraveineuse.

6. Combinaison pour l'utilisation selon la revendication 2 à 5, dans laquelle le cancer est le cancer de l'estomac ou le cancer du poumon.

7. Combinaison pour l'utilisation selon les revendications 2 à 6, dans laquelle le cancer est le cancer du poumon.

8. Combinaison pour l'utilisation selon la revendication 7, dans laquelle le cancer est le cancer du poumon non à petites cellules.

9. Kit, comprenant une quantité efficace d'emibetuzumab en association avec une quantité efficace du composé merestinib, ou d'un sel pharmaceutiquement acceptable de celui-ci, pour l'utilisation simultanée, séparée, ou séquentielle dans le traitement de cancer avec des tumeurs portant une omission d'exon 14 de MET ou un phénotype à omission d'exon 14 de MET.

10. Kit pour l'utilisation selon la revendication 9, dans lequel le cancer est le cancer de l'estomac ou le cancer du poumon.

11. Kit pour l'utilisation selon les revendications 9 ou 10, dans lequel le cancer est le cancer du poumon.

12. Kit pour l'utilisation selon la revendication 11, dans lequel le cancer est le cancer du poumon non à petites cellules.
